# EUROPEAN PATENT SPECIFICATION

(11) **EP 2 934 612 B1**
(45) Date of publication and mention of the grant of the patent: **15.03.2017**
(21) Application number: 13821692.4
(22) Date of filing: 20.12.2013
(51) Int. Cl.: A61L 24/10

(54) **SEALANT COMPOSITIONS**
DICHTUNGSMITTELZUSAMMENSETZUNGEN
COMPOSITIONS D'AGENT D'ÉTANCHÉITÉ

(30) Priority: 21.12.2012 EP 12198839
(43) Date of publication of application: 28.10.2015
(73) Proprietor: Thrombotargets Europe, S.L., 08860 Castelldefels - Barcelona (ES)
(72) Inventor: RODRÍGUEZ FERNÁNDEZ - ALBA, Juan Ramon, E-28100 Alcobendas, Madrid (ES); MURAT MORENO, Jesús, E-08860 Castelldefels (ES)
(74) Representative: ZBM Patents - Zea, Barlocci & Markvardsen
(86) International application number: PCT/EP2013/077674
(87) International publication number: WO 2014/096354

(56) References cited:
- EP-A1- 1 749 536
- WO-A1-97/29792
- WO-A1-99/18931
- WO-A1-2008/080989
- WO-A1-2011/131658

## Description

The present invention is in the field of sealant compositions.

### BACKGROUND ART

The set of physiological mechanisms triggered to stop bleeding after an injury is known as hemostasis. Hemostasis is a very complex process that involves a variety of molecules and cell types. To facilitate its study, hemostasis can be subdivided into four overlapping steps: i) vasoconstriction, ii) formation in the area of the wound of an initial platelet plug, iii) formation of a fibrin resistant clot (clotting cascade), and iv) a clot dissolution step or fibrinolysis, from which the healing process of the wound starts.

The extrinsic pathway of the coagulation cascade is initiated upon interaction of Factor VII (FVII), in its soluble and inactive form in plasma, with its specific receptor known as tissue factor (hereinafter also referred as "TF"). In its native form, TF is located predominantly on the surface of various cell types surrounding blood vessels. Therefore, under normal conditions, it contacts uniquely with plasmatic molecules in the case of an injury or wound. After the rupture of a blood vessel, the coagulation cascade is initiated by the association of TF with FVII, triggering the subsequent interaction between the various blood coagulation factors. The end result is the formation of thrombin (FIIa) that, in turn, converts the fibrinogen (FI), which is very abundant in plasma, into fibrin fibers. These fibrin fibers are insoluble, and form a mesh, which, together with platelets, results in what it is known as a fibrin clot.

With the aim of facilitating a rapid hemostasis in cases of massive hemorrhage, various procedures have been used over centuries, such as direct pressure, packing with gauze, suture-like threads, and tourniquet. These techniques, although effective, are known as "non-active" hemostatic means, and assist in the hemostatic process when used with different types of dressings.

In an attempt to improve surgical hemostatic settings, during the past decades a strong effort has been done in developing new products containing "active" ingredients which, in addition to traditional techniques, could help in the improvement of hemostasis. In this regard, the tissue glues, or fibrin sealants (hereinafter also referred as FS), represent new strategies for an industry of hemostatic/adhesive products that is rapidly evolving. The concept of using a FS to seal the edges of wounds is a relatively new idea. In fact, the FDA approved the use of the first accepted FS (Tisseel, Baxter) in 1998. Since then, commercially prepared fibrin sealants, such as Tiseel (Immuno, Vienna, Austria), Beriplast (Behringwerke AG), Tissucol (Baxter), and Biocol (CRTS) have been used extensively worldwide for almost 15 years.

FSs have many surgical applications and consist, primarily, of FI, FIIa, calcium chloride, and, occasionally, activated Factor XIII (FXIIIa). In some preparations, or in selected indications, an antifibrinolytic agent is included (aprotinin, tranexamic acid), to prevent lysis of the clot. The FSs are designed to mimic the final stage of the coagulation cascade during which, due to the catalytic action of the FIIa, FI is transformed into fibrin. The fibrin fibers formed become in close association with platelets, and give rise to the formation of the fibrin clot. In such interaction, FXIIIa plays an important role, since its presence allows the arrangement of the resulting fibrin network to acquire the optimum structural conformation in terms of resistance and elasticity.

The FSs provide FI and FIIa in much higher concentrations than they are present naturally in blood, thereby allowing a fast formation of a solid clot. Therefore, they polymerize independently of other blood components, and even work best in the absence of blood.

Since polymerization of FI and FIIa is very fast (less than 5 seconds), both components should be maintained in separate vials until their use. Therefore, marketed FSs are presented into two independent syringes which contents are mixed at the site of application

The mechanism of action of FSs is supported by the creation of a strong clot acting as a sealing barrier at the application site. FSs are indicated to be used over wounds areas wherein a loss of body fluids such as blood, bile, etc. occurs. They can also be used for the prevention of air leakage in lung wounds. In addition to their function as sealant agents, FSs could be used in various medical applications like: i) matrix for wound healing, ii) substrate for tissue adhesion, and iii) drug delivery.
It is well-known in the state of the art that a sealant composition has to be dual, showing both a hemostatic and sealant activity.
In contrast to conventional FSs consisting in dual isolated components (FIIa and FI), WO97/29792 describes a FIIa-free single-component sealant composition FI. The combination of TF and FI does not induce the formation of a stable clot. Thus, in addition to TF and FI, several coagulation factors are needed. This document discloses the need of including FII, FV, FVII, FX and FXIII to induce a clot. Therefore, in order to be effective, the sealant composition described in WO97/29792 is applied over healthy blood, or alternatively should also contain the missing coagulation factors (FII, FV, FVII, FX and FXIII) at physiological concentrations.
In spite of the efforts made, therefore, there is still the need of further compositions able to effectively prevent fluid migration from or into a tissue, which shows both an appropriate hemostatic and sealing activity.

### SUMMARY OF THE INVENTION

The inventors of the present invention have found that by adding a lipidated TF to already known FS compositions, comprising both thrombin and fibrinogen, there is a substantial improvement in the hemostatic profile. In addition, the clot resulting from the application of the combination of the invention shows a higher consistency, which is indicative of its efficiency in sealing a bleeding wound/injury. This means that a combination comprising at least thrombin, fibrinogen and a lipidated TF, is a promising sealant composition which overcomes/improves the hemostatic/sealing dual effect of the already known FS compositions.
This substantial improvement in the hemostatic profile is mainly due to the propagation capability of the clot combination nearby its site of application. This propagation effect is extremely important not only in healthy blood, but also in blood in which the activity of crucial hemostatic components such as FIIa and FX is impaired by the administration of certain drugs widely used in surgery, such heparins. Unexpectedly, the clot propagation observed with the combination of the invention overcomes the anticoagulative stage of blood treated with heparins.
None of the prior art document disclosing sealant compositions describes their efficacy on the basis of the propagation capability of the clot to the area nearby its site of application.

This means that a combination comprising at least thrombin (FIIa) fibrinogen (FI) and a lipidated TF, is a promising sealant composition which overcomes/improves the hemostatic/sealing dual effect of the already known FS compositions.
Thus, in an aspect the present invention provides a combination comprising a lipidated tissue factor, thrombin, and fibrinogen.

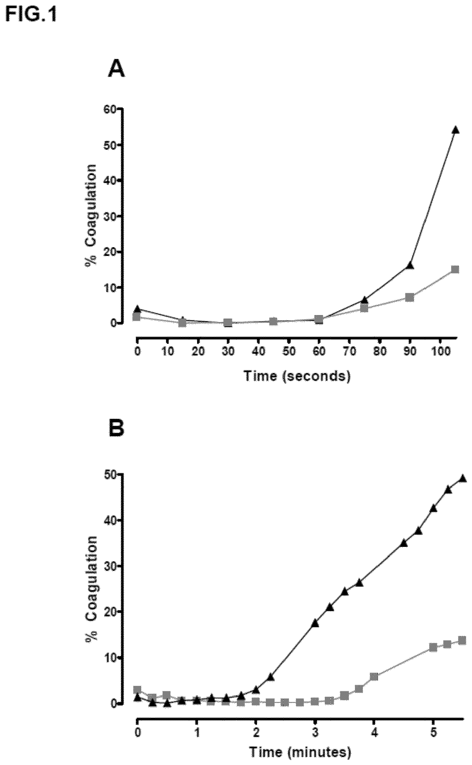

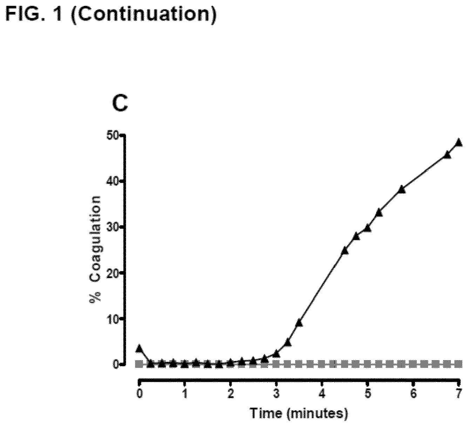

In FIG. 1 (A) it is shown that when a lipidated tissue factor is added to a marketed sealant composition, Tissucol®, (comprising thrombin and fibrinogen), the induced coagulation in plasma (through the propagation of the clot in healthy plasma at a distance of 3 cm away from the site of application) takes place in a period of time substantially lower than the one needed when only Tissucol® is used. In fact, as it can be derived from said data, when the coagulation is of about 50% with the combination of the invention (black triangle), the combination of the prior art (Tissucol®, grey square) starts to coagulate the plasma sample. It is remarkable, and firstly described herein, that this difference is even greater in the case of heparin treated plasma at the lower therapeutically dose (FIG. 1 B) and the difference is maximal when the higher therapeutically dose of heparin was used (FIG. 1 C). In this later case, the heparin-treated plasma did not even start to coagulate at 7 min. after FS application. Thus, by adding a Z lipidated TF to a FS comprising FIIa and FI a substantial improvement in the hemostatic profile is achieved.
This means that administering the combination described in the invention, much less time is required to stop bleeding. This is of special relevance in cases of heparin treated patients, wherein compressions with thrombin or other hemostatic products are not efficient enough to stop bleeding, being a high risk that the patient dies.

In addition, the present inventors have found that the combination comprising fibrinogen, thrombin, and a tissue factor shows a surprising sealant effect. As it is explained below, in Example 4, a combination comprising thrombin, a lipidated tissue factor, and fibrinogen, when applied to a heparinized plasma sample, spreads substantially faster through it than a sealant combination not including the lipidated TF. In addition, it has been observed that the clot obtained with the combination including a tissue factor, thrombin, and fibrinogen has a much higher consistency than the clot obtained with Tissucol® (i.e., not including lipidated tissue factor). Without being bound to the theory, the present inventors believe that the tissue factor is embedded in the fibrin network formed (due to the thrombin and fibrinogen included in the combination) and that due to this distribution of the tissue factor within the fibrin network, an expansion effect of the clot occurs, giving rise to the efficiently sealof the injury or lesion. The fact that the clot obtained with the combination of the invention shows a better consistency with respect to the one obtained with Tissucol® means that the combination of the invention is more effective in preventing the blood migration, that is more effective in sealing the injury/wound, reducing substantially the risk of clot breakage.
It is remarkable that spreading of the clot formed by the ternary composition of the present invention in both, normal and heparin-treated plasmas, can not be anticipated by the teachings of WO97/29792. In particular, this document discloses thrombin-free sealant compositions including thromboplastin. In said document, page 6, lines 6-8, it is stated that the inclusion of thromboplastin as the initiator of fibrin clot formation may improve the hemostatic qualities of the adhesive. However, the experimental data provided in Example II (also summarized in Table VI) show that the time required to hemostasis is substantially the same or even higher than the time required for prior art tissue adhesives. Which means that it is equal or worse hemostatic agent than the prior art compositions. Therefore, the experimental data provided in WO97/29792 would not motivate to the skilled person in the art to add lipidated tissue factor to a composition comprising thrombin and fibrinogen in order to obtain a sealant composition with improved hemostatic properties. On the contrary, from the teachings of this document the skilled person would not expect any substantial improve in the hemostatic effect of a composition with the addition of thromboplastin.

The inventors of the present invention have tried to reproduce the content of WO97/29792 in order to determine which the effect of lipidated tissue factor is when it is added to a free-thrombin sealant composition. As it is summarized in Table I below, the compositions of WO97/29792 show very low MCF and G values, when compared to those of some commercial sealant compositions. As the skilled in the art knows, as higher the G and MCF values are, as best hemostatic and sealant profiles has the composition. Therefore, from the results summarized in Table I below, the skilled person would conclude that the inclusion of lipidated tissue factor in a thrombin-free sealant composition, as disclosed in WO97/29792, would not improve the hemostatic profile of the such composition.

Finally, WO97/29792 is silent about the effect of adding a lipidated tissue factor on the sealant profile of thrombin sealant compositions.
In conclusion, from WO97/29792 the skilled person would not have the motivation of adding a lipidated tissue factor to a sealant composition with the expectation of achieving a substantial improvement in the hemostatic and sealant profiles.
Surprisingly, the inventors of the present invention, as it has been stated above, have found that including a lipidated tissue factor in a composition comprising thrombin and fibrinogen, there is achieved a substantial improvement in the hemostatic and sealant profiles.
Therefore, due to the excellent hemostatic and sealant properties of the combination comprising thrombin, fibrinogen, and lipidated tissue factor, such a combination can be used in the formulation of a sealant composition together with an appropriate carrier material.
Thus, in a second aspect the present invention provides a sealant composition comprising the combination as defined in the first aspect of the invention, and a material carrier.
In a third aspect the present invention provides the use of the combination of the first aspect of the invention as a sealant agent.

Due to the properties of the combination and sealant composition of the invention, they can be used in the form of pharmaceutical or veterinary compositions.

Therefore, in a fourth aspect the present invention provides a pharmaceutical or veterinary composition comprising a therapeutically effective amount of the combination as defined above, together with other appropriate pharmaceutically or veterinary acceptable excipients and/or carriers.

As it has been discussed above, the combination and composition of the invention, due to its hemostatic and sealant effects, are useful in the treatment of hemorrhages.

Thus, in a fifth aspect the present invention provides the combination or composition as defined above for use as a medicine. This aspect can be formulated as a method for the treatment of a disease comprising administering to a subject in need thereof a therapeutically effective amount of the combination or composition as defined above together with other appropriate pharmaceutically or veterinary acceptable excipients and/or carriers.

In a sixth aspect the present invention provides a combination or a composition as defined above for use in the treatment of haemorrhages.

This aspect can alternatively be formulated as the use of a combination or composition as defined above in the manufacture of a medicament for the treatment of hemorrhages. This aspect can be alternatively formulated as a method for the treatment of hemorrhages, the method comprising administering to a subject in need thereof of a therapeutically effective amount of the combination or composition of the invention.

The frequency of use of marketed FSs as adhesive or sealant in several surgery settings, has open the possibility of their use as carrier for the slow release of relevant drugs.

In this regard, several FSs containing antibiotics have been tested to reduce the postoperative ocular infections, and for the treatment of localized peritoneal infections. It has been found that such compositions improve the therapeutic effect of the active substance due to the prolonged time that they remain adhered on the site. Thus, combinations and compositions of the invention comprising thrombin, fibrinogen, and a lipidated TF containing also other active molecules, such as antibiotics, growth factors, chemotherapeutics agents, topical anesthetics, or DNA-based vectors could provide an increased therapeutic effect.
In a further aspect, therefore the present invention provides the use of the combination or composition of the present invention as a drug delivery system.
It has also been described in the state of the art the use of FS to assist with the construction of skin, cartilages, and bones. Therefore, the proposed combination of FS plus lipidated TF could also be used as an improved scaffold for cellular attachment and growth of new tissue. This potential could be amplified by the well known angiogenic effect of TF.
Thus, in a further aspect the present invention provides the use of the combination or composition of the present invention as wound healing agent. Finally, the combination of the invention or any of the compositions of the second and third aspects of the invention, can be provided as a kit.
Thus, in a further aspect the present invention provides a kit comprising the combination as defined in the first aspect of the invention, and an applicator that allows the simultaneous application of both fibrinogen, thrombin, and lipidated tissue factor.

### BRIEF DESCRIPTION OF THE DRAWINGS

FIG. 1 shows the progression of coagulation in normal plasma (A), or in heparin treated plasma (B and C) samples at different times after adding the FS composition marketed under the trademark Tissucol®, (grey square) or a combination of lipidated TF and Tissucol® (black triangle). Coagulation was determined 3 cm away from the site of application of the test item. Y-axis= % of coagulation; X-axis= time (A seconds, B and C minutes).

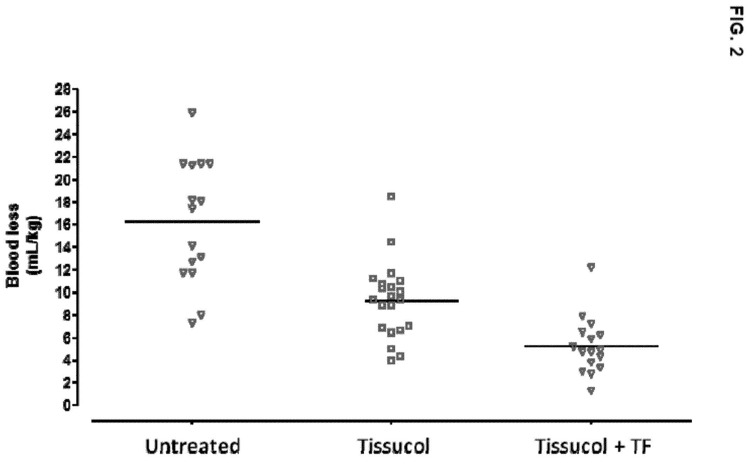

FIG. 2 represents the blood loss in mL/Kg for each rat corresponding to the groups treated with the different products: Tissucol ® (FS), Tissucol ® + lipidated TF, and control (untreated). Each point (triangle or square) represents the blood loss value of a single animal, and each of the horizontal lines represents the average of each group.

### DETAILED DESCRIPTION OF THE INVENTION

As it has been stated above, the combination of the present invention contains thrombin, fibrinogen, and lipidated tissue factor.
In the present invention, the term "tissue factor" (TF) has to be understood as an integral membrane glycoprotein that is widely distributed in the animal kingdom. TF appears in different cells types, predominantly in the subendothelial tissue, and is necessary for the initiation of the extrinsic pathway of coagulation cascade leading to the production of FIIa and the formation of a stable fibrin clot. Exemplary TF proteins that can be used in the present invention include human TF (UniProtKB/Swiss-Prot. Version 148, November 2012, accession number P13726), mouse TF (UniProtKB/SwissProt. Version 103, November 2012, accession number P20352), bovine TF (UniProtKB/Swiss-Prot. Version 89, November 2012, accession number P30931), rabbit TF (UniProtKB/Swiss-Prot. Version 90, November 2012, accession number P24055) and TF proteins of different animals.
The human TF protein with the SwissProt accession number P13726 has a well-defined domain structure which comprises (1) a signal peptide or a region with a 32 amino acid leader sequence that is post-translationally processed from the immature to the mature form; (2) an N-glycosylated hydrophilic extracellular domain comprising 219 amino acids; (3) a highly hydrophobic fragment of 23 amino acids, which form the transmembrane domain; and (4) the 21-amino acid carboxyl end which is the protein cytoplasmic fragment.
In a particular embodiment, the TF corresponds to the mature TF.
The term "mature TF" as used herein, refers to the TF protein whose amino acid sequence lacks the signal peptide. In an embodiment, said mature TF protein is the mature human TF of SEQ ID NO: 1.
In one embodiment, the TF is glycosylated.

As used herein, the term "glycosylated" includes any degree of glycosylation. Since native TF contains several glycosylation sites, as explained in detail below, the expression "tissue factor" also encompasses those forms of the protein showing different degrees of glycosylation, said forms being obtained by expressing TF in hosts capable of carrying out N-glycosylation reactions.

In addition, the term "tissue factor" also embraces those variants resulting from the insertion, deletion, or substitution of one or more amino acid residues in the native TF sequence. Suitable functional assays that can be used to assess whether a given polypeptide is a functional variant of TF are those assays based on the determination of the ability of the TF variant to specifically bind FVIIa, or on the *in vitro* determination of the coagulation time in plasma or whole blood, by an *in vivo* assay in a severe hemorrhage animal model or by an *in vivo* assay in a lethal hemorrhage animal model. Procedures for carrying out these assays have been disclosed in the prior art.

Such variants according to the present invention include amino acid sequences that are at least 60%, 70%, 80%, 90%, 95% or 96% identical to the native TF molecules mentioned above. As known in the art the "identity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm.

The TF may be fully glycosylated, partially glycosylated or non-glycosylated.

In the present application "fully glycosylated" means that all potential glycosylation sites have been glycosylated.

In the present application "partially glycosilated" means that at least one of the three N-glycosylation sites is non-functional, being not possible its glycosilation.

In the present application "non-glycosylated" means that none of the N-glycosylation sites is functional, or alternatively, that the TF polypeptide has been expressed by a non-glycosilatyon method in vitro (i.e. cell-free expression system), or in a vector deprived of metabolic glycosylation pathways (i.e. *E. coli*)*.*

In an embodiment, the TF is partially glycosylated or non-glycosylated. In this embodiment, the TF has been modified in such a way that at least one of the N-glycosylation sites is non-functional, being not possible its glycosylation.

All mature TF proteins contain three potential N-linked glycosylation sites having the consensus sequence Asn-Xaa-Ser/Thr. In the case of human mature TF, such three glycosilation sites are located at Asn11 (sequence Asn11-Leu12-Thr13), Asn124 (sequence Asn124-Val125-Thr126) and Asn137 (sequence Asn137-Asn138-Thr139), said positions being in respect of SEQ ID NO: 1.

In an embodiment, the N-glycosylation site or sites which can be modified to make them non-functional in the human mature TF protein are those corresponding to the N-glycosylation sites NLT at positions 11-13, NVT at positions 124-126, and NNT at positions 137-139 in the mature human TF of sequence SEQ ID NO: 1 above.

In another embodiment, the TF carries one or more substitutions of the Asn residues into residues which are not acceptors for N-glycosylation. In a still more preferred embodiment, the TF variant comprises one or more Asn-to-Ala mutations in the Asn residues in positions corresponding to positions 11, 124 or 137 in the mature human TF. Preferably, the mature human TF carries the mutation Asn to Ala at position 124 of SEQ ID NO: 1.

The glycosylation will vary depending on the expression system used for the production of TF. For instance, the tissue factor protein can include one or more plant-specific glycan, yeast-specific glycan, insect-specific glycan, or animal-specific glycan.

When TF is produced in yeast, the glycosylation typically will involve an inner core of about ten mannose residues, linked to the asparagine via two GlcNAc residues, and a branched outer chain of 50-100 mannose residues. Therefore the N-linked glycosylation could potentially add as many as 300 mannose residues to TF, an increase in molecular mass in about 60 kDa. Furthermore, it is also possible that several mannose residues could be attached to various O-linked glycosylation sites (more than 25). Therefore, TF molecules included in the combinations and compositions of the present invention have a variable degree and composition of N-linked glycosilation in one or more N-glycosylation sites.

In still another embodiment, the TF protein is a fusion protein, said fusion protein comprising a first portion, which comprises said TF protein, and a second portion, which comprises another peptide or protein.

Said second portion may be bound to the N-terminus first portion of said TF protein fragment, or, alternatively said second portion may be bound to the C-terminus region of said TF protein fragment. Both first and second portions may be directly bound or bound through a linker polypeptide between said first and second regions.

In another embodiment, said second portion is a tag. The tag can be bound to the C-terminal or N- terminal domain of the TF protein first portion.
In the present invention, the term "tag" has to be understood as any peptide or amino acid sequence, bound to the C-terminal or N-terminal domain of said TF protein, which can be used in the isolation or purification of the fusion protein. Thus, said tag is capable of binding to one or more ligands, such as, for example, one or more ligands of an affinity matrix such as a chromatography support or bead with high affinity. An example of said tag is a histidine tag (His-tag or HT), such as a tag comprising 6 residues of histidine (His6 or H6), which can bind to a column of nickel (Ni²⁺) or cobalt (Co²⁺) with high affinity. Additional illustrative, non-limitative, examples of tags useful for isolating or purifying a fusion protein include Arg-tag, FLAG-tag, Strep-tag, an epitope capable of being recognized by an antibody, such as c-myc-tag (recognized by an anti-c-myc antibody), SBP-tag, S-tag, calmodulin binding peptide, cellulose binding domain, chitin binding domain, glutathione S-transferase-tag, maltose binding protein, NusA, TrxA, DsbA, Avi-tag, among others, an amino acid sequence such as Ala-His-Gly-His-Arg-Pro (SEQ ID NO:2); Pro-Ile-His-Asp-His-Asp-His-Pro-His-Leu-Val-Ile-His-Ser (SEQ ID NO:3); Gly-Met-Thr-Cys-X-X-Cys (SEQ ID NO:4); β-galactosidase and the like.

It has been previously disclosed that His-tag, used for the isolation or purification of recombinant TF, has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Thus, in one embodiment, the tag is a His-tag. In another embodiment, such His-tag is bound to the C-terminal domain of the TF protein first portion. In still another embodiment, such His-tag is bound to the N-terminal domain of the TF protein first portion.

In another embodiment, the first portion of the fusion protein comprises the mature TF protein, preferably, the mature human TF protein. In still another embodiment, the first portion is the human mature TF.

In another embodiment, the fusion protein has a first portion which comprises the mature human tissue factor protein with at least one of the N-glycosilation sites being non-functional, and a second portion which comprises a His-tag. In a preferred embodiment, the tissue factor is a fusion protein comprising a human TF of SEQ ID NO: 5 which: (a) lacks the signal sequence, (b) has a N124A mutation at the glycosilation site, (c) has an hexahistidine tag at the C terminus.

Said fusion protein may be obtained by conventional means, e.g., by means of gene expression of the nucleotide sequence encoding for said fusion protein in a suitable yeast cell. The eventual tag can be used, if desired, for the isolation or purification of said fusion protein.

The term "lipidated Tissue Factor (TF)" as used herein refers to any source of TF being this TF totally or partially inserted in lipidic vesicles or cellular membranes. Illustrative, non-limiting examples of "lipidated TF" sources are: 1) lipidated TF containing tissue extract (whose isolation can be carried out from several tissues such as cerebral, placental and lung tissue, tissues from different animals such as sheep, cows, rabbits, dogs, and human beings, among others); 2) purified and (re)-lipidated TF proteinaceous component, i.e. what the lipid component (phospholipids) has been added to after TF purification; and 3) lipidated TF containing cell extract where lipidic fraction comes from the host cell and TF has been recombinantly expressed.

Purified and (re)-lipidated TF can be prepared, by way of an illustrative, non-limiting example, according to the protocol previously described by Mimms L. T. et al., "Phospholipid vesicle formation and transmembrane protein incorporation using octyl glucoside", Biochemistry, 1981, vol. 20(4), p. 833-840. In said protocol, non-lipidated TF is incorporated within phospholipid vesicles using a non-ionic detergent, such as N-octyl-beta-D-galactoopyranoside, for example. The phospholipids that can be used in lipidated TF according to the invention may have any origin (animal, plant or synthetic). Virtually any phospholipid can be used in the preparation of the lipidated TF of this invention. Illustrative, non-limiting examples of phospholipids that can be used in the preparation of lipidated TF include phosphatidylcholine, phosphatidylserine, phosphatidylethanolamine, etc. The TF proteinaceous component:phospholipid ratio (molar, weight or volumetric) may vary within a wide range, for example, from about 1:50000 to about 1:3000. In a particular embodiment the lipidated TF is a lipidated human TF and consists of the proteinaceous component of a TF isolated from human tissue, or from recombinant cells, and inserted in a lipid membrane in a suitable TF proteinaceous component:phospholipid ratio. The preferred molar ratios of lipid composition (preferible phosphatydylcholine and phosphatydylserine) to achieve the optimal TF activity is well known in the state of the art.

Concerning the preparation of lipidated TF containing cell extract where lipidic fraction comes from the host cell and TF has been recombinantly expressed,since the TF molecule contains a large hydrophobic domain, it is reasonable to assume that cloning of rTF gene in any of the available eukaryotic expression systems would result in the expression or recombinant TF (rTF) protein associated to host cells lipidic membranes. In this association it would be possible that host membranous components could mimic or provide similar or identical scaffold to the rTF molecules as those found in mammalian cells to witch TF normally associates. Thus, purification of these mermbranous components would result in an available source of lipidated rTF. Due to its chemical composition, these isolated lipidic membranous components would be found predominantly in the form of vesicles of different sizes. In addition to rTF those vesicles would also contain specific proteins or lipids depending on the origin of the host cell. Therefore, the lipidic membranes coming from yeast, bacteria, cells from different origin such as insect, mammal, plant, fish, algae, would contain also proteins and lipids characteristics of such host cells).

TF comprises a hydrophobic region (the transmembrane domain) which is anchored in a lipid membrane, whereas the hydrophilic regions thereof (i.e., the amino-terminus region and the carboxyl-terminus region of said TF protein) face the exoplasmic side of the membrane.

In the present invention, the term "lipid membrane" has to be understood as an organized layer of a few molecules (lipids and proteins) thick forming the boundary of a cell (i.e., the cell or plasma membrane) or the boundaries of intracellular organelles. Typically a membrane is composed of two oriented lipid layers in which proteins can be embedded. A lipid bilayer, which is the basic structure of the membranes of a cell, is usually formed by amphipathic molecules (e.g. phospholipids, fatty acids etc.) in an aqueous environment, each molecule being oriented with the hydrophilic group on the outside of the layer and the hydrophobic group to the interior of the layer. Preferably, the TF is anchored in a lipid microvesicle.

In the present invention, "lipid microvesicle" has to be understood as a small and closed compartment, which is substantially composed by lipids mono or bilayers. The size of the TF-bearing microvesicle of the invention can vary within a relatively broad range, usually, said size is equal to or lower than 10 µm, typically equal to or lower than 0.5 µm. In a particular embodiment, the size of the TF-bearing yeast derived microvesicles of the invention range from 10 to 0.01 µm. The microvesicles are formed by lipid membranes, or fragments thereof, from eukaryotic cells. In one embodiment, the microvesicle can be enriched in negatively-charged phospholipids, preferably, phosphatidilserine. Methods for enriching such microvesicles in phosphatidilserine are disclosed, for instance, in WO2011131658. Briefly, said TF-bearing microvesicles enriched in phospholipids can be prepared by a process comprising: a) subjecting a culture of recombinant eukaryotic cells expressing TF protein to fermentation under conditions which allow the expression of said TF protein, or fragment thereof having pro-coagulant activity; b) pelleting the cultured cells resulting from the fermentation of step a), to render a fermentation product; c) subjecting said fermentation product from step b) to homogenization, to render a fermentation homogenate; and d) subjecting said fermentation homogenate from step c) to separation, to render a pellet and a clarified yeast extract (CYE) containing said TF-bearing derived microvesicles having pro-coagulant activity; e) collecting said clarified yeast extract (CYE) containing said TF-bearing yeast derived microvesicles having pro-coagulant activity; and, optionally, f) if desired, isolating or purifying said TF-bearing yeast derived microvesicles having pro-coagulant activity by size partitioning procedures, and g) enrichment of the obtained microvesicles in negatively charge phospholipid (i.e. phosphatydilserine) by the incubation of both components phosphatidylserine and lipid microvesicles containing TF as described in WO2011131658. As a step previous to the mentioned incubation, phosphatydilserine could be prepared as multilamelar vesicles by resuspension of lipids in a buffered solution followed by sonication.

The method of production of TF depends on the eukaryotic cell used. Generally, the eukaryotic cell is transformed with a expression vector comprising the nucleotide sequence coding for TF protein operatively linked to a functional promoter in a cell which can be from: fungi, yeast, plant or animal (such as fish, reptilian, mammalian, insect, etc). The cDNA coding for TF protein can be amplified by the polymerase chain reaction (PCR) using a cDNA library as template and the appropriate primers. Example 1 discloses the amplification of the cDNA coding for the mature hTF protein with 18 extra nucleotides (coding for six histidines) at the 3' end.

In another embodiment, the TF protein could be purified from steps a) to d) above, following well established chromatography purification procedures. Once purified, the TF protein could be lipidated in vitro with optimal phospholipid concentrations.

In an additional embodiment, lipidated TF could be obtained from animal tissue extracts, such as cerebral, placental and lung tissue,and tissues from different animals such as sheep, cows, rabbits, dogs, and human beings, among others. Preferably, the lipidated TF is from human being. More preferably, the lipidated TF is human recombinant.

In the present invention, the term "thrombin" (FIIa) has to be understood as the key serine protease that plays a pivotal role in hemostasis. FIIa activates many of the coagulation factors involved in the coagulation cascade including Fibrinogen (FI), Factor V (FV), Factor VIII (FVIII), Factor XI (FXI), and Factor XIII (FXIII). FIIa also activates platelets and vascular endothelial cells. The thrombin precursor is the zymogen Prothrombin (FII), which is secreted into blood from the liver. During the coagulation cascade, FII is converted into FIIa at different rates first by activated FX (FXa), and at the latest steps of the cascade by the prothrombiase complex (FXa:FVa).Commercial FIIa is produced after isolation of FII from plasma of human or bovine origin. After its isolation, FII is transformed in vitro into FIIa. All current marketed FSs contain FIIa from human or bovine origin. Additionally, highly purified recombinant human FIIa is also available. This recombinant human FIIa is obtained after purification of human FII from recombinant Chinese Hamster Ovary (CHO) cells, followed by its processing to FIIa by in vitro procedures.

Exemplary FII proteins that can be used in the present invention include human FII (UniProtKB/Swiss-Prot. Version 182, November 2012, accession number P00734.), bovine FII (UniProtKB/Swiss-Prot.Version 153, November 2012, accession number P00735), mouse FII (UniProtKB/Swiss-Prot. Version 141, November 2012, accession number P19221), pig FII (UniProtKB/SwissProt. Version 37, November 2012. accession number B3STX9), and FII proteins of different animals. Preferably, the thrombin is from human or bovine origins, although more preferably is human thrombin and even more preferably recombinant human thrombin.

In the present invention, the term "fibrinogen" (FI) corresponds to the penultimate protein in hemostasis. FI shows a complex structure, since it consists of two α (66kDa), two β (52 kDa), and two γ (46.5 kDa) chains, held together by 29 disulfide bonds. FI is synthesized in the liver, and circulates in plasma at a concentration of 2-4 mg/mL. During the last step of the coagulation cascade, FI is activated to Fibrin by FIIa. Fibrin aggregates forming a cross-linked network of fibers, which in collaboration with platelets forms the clot. Current FSs contain plasma-derived fibrinogen, which is purified from human blood. This procedure has associated risks of pathogen contamination. These risks could be minimized by producing a recombinant version of human fibrinogen. Recombinant human FI has been produced in *E coli,* Baby Hamster Kidney (BHK) cells, Monkey Kidney (COS-1) cells, and CHO cells. Recently, human FI has been produced in transgenic cows, from where recombinant FI is obtained from the milk.

Exemplary FI proteins that can be used in the present invention include human FI: α chain (UniProtKB/Swiss-Prot. Version 174, November 2012, accession number P02671), β chain (UniProtKB/Swiss-Prot. Version 163, November 2012, accession number P02675), γ chain (UniProtKB/Swiss-Prot. Version 171, November 2012, accession number P02679); bovine FI: α chain (UniProtKB/Swiss-Prot. Version 104, November 2012, accession number P02672), β chain (UniProtKB/Swiss-Prot. Version 102, November 2012, accession number P02676), and FI proteins of different animals. Preferably, the fibrinogen included in the combination of the invention is of human origin, and more preferably is recombinant human FI.

In one embodiment of the first aspect of the invention, the combination further comprises a cross-linking agent.

In another embodiment of the first aspect of the invention, the combination further comprises a fybrinolysis inhibitor.

In still another embodiment of the first aspect of the invention, the combination further comprises CaCl₂.

In a further embodiment of the first aspect of the invention, the combination further comprises a cross-linking agent and a fybrinolysis inhibitor.

In a further embodiment of the first aspect of the invention, the combination further comprises a cross-linking agent, fybrinolysis inhibitor, and CaCl₂.

Preferably, said cross-linking agent is FXIII.

Preferably, said fybrinolysis inhibitor is selected from aprotinin, tranexamic acid, and aminocaproic acid.
In a further aspect, the present invention provides hemostatic sealant compositions comprising the combinations of the invention and a carrier material.

In the present invention, the term "hemostatic" referred to compositions, effect and use, has to be understood as a substance that promotes hemostasis, that is, that stops bleeding. It can also be referred as "antihemorrhagic" and "procoagulant".

The term "carrier material" has to be understood as a substrate of suitable material allowing depositing the combinations of the invention thereon, its transport and its release at the desired site, for example, in the site where the compositions of the invention has to exert its therapeutic effect. Said carrier material can be a solid support or a non-solid support, for example, a liquid support, a powdered support or a gaseous support. Illustrative, non-limiting examples of solid supports include dressings, band-aids, compresses, plasters, etc. Illustrative, non-limiting examples of liquid supports include gels, sprays, mouthwashes, etc. Illustrative, non-limiting examples of gaseous supports include air, propellants, etc. The manufacture of such compositions can be obtained by conventional methods, for example, by mixing the combinations of the invention and the material carrier. The interaction between the components of the combination and the solid material can be a physical or chemical interaction.

In one embodiment, the material carrier is a solid support made of collagen, gelatin or oxidized regenerated cellulose.

In another embodiment, the carrier material is spray, an aerosol, or a device for the dispensing of a powder.

In one embodiment of the second aspect of the invention, the sealant composition further comprises a cross-linking agent.

In another embodiment of the second aspect of the invention, the sealant composition further comprises a fybrinolysis inhibitor.

In still another embodiment of the second aspect of the invention, the sealant composition further comprises CaCl₂.

In a further embodiment of the second aspect of the invention, the sealant composition further comprises a cross-linking agent and a fybrinolysis inhibitor.

In a further embodiment of the second aspect of the invention, the sealant composition further comprises a cross-linking agent, fybrinolysis inhibitor, and CaCl₂.

Preferably, said cross-linking agent is FXIII.

Preferably, said fybrinolysis inhibitor is selected from aprotinin, tranexamic acid, and aminocaproic acid.

As mentioned above, in a further aspect the present invention provides a pharmaceutical or veterinary composition comprising a therapeutically effective amount of the combinations of the invention, together with appropriate pharmaceutically or veterinary acceptable excipients and/or carriers.

The expression "therapeutically effective amount" as used herein, refers to the amount of the combination that, when administered, is sufficient to prevent development of, or alleviate to some extent, one or more of the symptoms of the disease which is addressed. The particular dose of the combinations administered according to this invention will of course be determined by the particular circumstances surrounding the case, including the compound administered, the route of administration, the particular condition being treated, and the similar considerations.

Processes for the preparation of such sealant compositions are well-known in the state of the art.

The pharmaceutical or veterinary composition of the invention should be applied topically to the bleeding site, either directly or in a carrier material, such as a gauze. Examples of topical application includes, but are not limited to, external, cutaneous, intranasal, intravesicular, intravaginal, buccal, rectal, ophthalmic, endoscopic topical application, and instillation. Preferably, the combination and compositions of the present invention are applied topically.

The pharmaceutical or veterinary composition can take the form of soft or hard gel, liquid, lotion, cream, ointments, pastes, roll-on formulations, sprays, aerosols, pad-applied formulations and film-forming formulations.

For the topical administration, appropriate pharmaceutical excipients or carriers include, but do not limit to, hydrating agents, emollients, emulsifiers, humectants, pH-regulating agents, antioxidants, preservative agents, vehicles, or mixtures thereof. The excipients or carriers used have affinity for the skin, are well tolerated, stable, and are used in an amount adequate to provide the desired consistency, and ease application.

When the pharmaceutical composition of the present invention is topical, it can be formulated in several forms that include, but are not limited to, solutions, lotions, gels, ointments, pastes, creams, of soft or hard gel, liquid, lotion, cream, ointments, pastes, roll-on formulations, sprays, aerosols, pad-applied formulations and film-forming formulations. These topical pharmaceutical compositions can be prepared according to methods well known in the state of the art. The appropriate pharmaceutical excipients and/or carriers, and their amounts, can readily be determined by those skilled in the art according to the type of formulation being prepared.

The compositions of the invention, can be used as an adjunct to hemostasis in patients undergoing surgery when control of bleeding by conventional surgical techniques (such as suture, ligature, and cautery), as well as adjunct in standard surgical techniques (such as suture and ligature) to prevent leakage.

In a further aspect, the invention provides the use of the combination of the fourth aspect of the invention as sealant agent.

As it has been stated above, the combination of the invention, due to the high consistency of the clot produced and the spread with which it expands can seal efficiently any injury or lesion in a very short period of time, it is prevented the fluid migration in a safety and effective way because the risk of breakage is substantially reduced. This application is of interest in sealing injuries or wounds in the skin, organs, and after an internal traumatism, among others.

In one embodiment, the combination or compositions as defined above are used to seal a tissue by suturing tissues together, when possible, and applying the combination or composition to the tissues.

In another embodiment, the combination or compositions as defined above are used to fix a graft to a tissue, by applying the combination or composition to the tissue prior to placing the graft on it.

Due to the properties pointed out in detail above, the combinations and compositions of the invention can be used as a drug delivery system or as a wound healing agent.

The expression "wound healing" relates to an intricate process in which the skin (or some other organ) repairs itself after injury wound healing of any kind and at any site. It can be normal and impaired wound healing. The latter is found in particular in the case of diseases, such as diabetes mellitus, vasculitis, arterial occlusive disease, chronic venous and/or infected ulcer as well as poorly healing gastric ulcer. Impaired wound healing is also found in the case of innervations impairment such as paraplegia, leprosy, neuropathy, etc., and decubital gangrene of persons in need of care. Impaired wound healing will also be given if weak sutures and impaired healing occur after operations, particularly of the intestines and transplantations of skin and other organs, respectively.

Impaired wound healing is also found in the case of bone fractures, burns and treatments using steroids.

As used herein, the term "wound" includes an injury to any tissue, including for example, delayed or difficult to heal wounds, and chronic wounds. Examples of wounds may include both open and closed wounds. The term "wound" may also include for example, injuries to the skin and subcutaneous tissue initiated in different ways (e.g., pressure sores from extended bed rest and wounds induced by trauma) and with varying characteristics. Wounds may be classified into one of four grades depending on the depth of the wound: i) Grade I wounds limited to the epithelium; ii) Grade II wounds extending into the dermis; iii) Grade III wounds extending into the subcutaneous tissue; and iv) Grade IV (or full-thickness wounds) wounds wherein bones are exposed (e.g., a bony pressure point such as the greater trochanter or the sacrum).

The term "chronic wound" generally refers to a wound that has not healed. Chronic wounds include venous ulcers, venous stasis ulcers, arterial ulcers, pressure ulcers, diabetic ulcers, diabetic foot ulcers, vasculitic ulcers, decubitus ulcers, burn ulcers, trauma-induced ulcers, infectious ulcers, mixed ulcers, and pyoderma gangrenosum.

The combinations and compositions of the invention can also be used in the treatment of a hemorrhages. Such hemorrhages can be due to coagulopathies, injuries, wound, or platelet disorders, among others.

As it is shown below, the combinations of the invention act as antihemorrhagic agent, and, consequently, can be used to treat or correct hemorrhagic disorders, particularly those hemorrhagic disorders associated with hemorrhagic diathesis.

The term "hemorrhagic diathesis" refers to the process causing a hemostasic disorder and which, as a result, gives rise to the occurrence of a hemorrhagic syndrome which may occasionally occur with extended and excessive bleeding.

The term "coagulopathy" refers to a coagulation factor disorder. This disorder may be due to a specific coagulation factor deficiency or deficit, the consequence of which will be the occurrence of a hemorrhagic syndrome, or due to a coagulation factor disorder. The coagulopathy may generally be a congenital coagulopathy or an acquired coagulopathy. As illustrative, non-limiting examples of congenital coagulopathies, deficiencies of coagulation factors selected from coagulation Factor V (FV), coagulation Factor VII (FVII), coagulation Factor VIII (FVIII), the deficit or deficiency of which causes hemophilia A, coagulation Factor IX (FIX) the deficit or deficiency of which causes hemophilia B, coagulation Factor X (FX), coagulation Factor XI (FXI) the deficit or deficiency of which causes hemophilia C, coagulation Factor XII (FXII), coagulation Factor XIII (FXIII) and their combinations, can be mentioned. Acquired coagulopathies may have different origins. Illustrative examples include coagulation factor synthesis deficiencies in severe hepatic failure, anticoagulant therapy (such as heparin, low molecular weight heparins, warfarin, coumarin derivatives, dicoumarins, etc.). An alternative mechanism is based on an exaggerated consumption of coagulation factors such that they are not available to form the clot in a bleeding lesion. This mechanism occurs in the disseminated intravascular coagulation syndrome or coagulopathy due to consumption occurring in multiple illnesses such as in severe sepsis damaging the microcirculation endothelium activating platelets and coagulation factors with the formation of multiple microthrombi; in blood invasion by TF such as placental release; in the retention of a dead fetus; in multiple traumas with the crushing of tissues; in poisonous snake bites, etc. In vasculitis, parietal and endothelial damage releases coagulation activators. The consumption of coagulation factors is worsened by lysis of the fibrin of numerous microthrombi due to the action of plasmin, which are antiplatelets and anticoagulants.

The term "platelet disorder" refers to a disorder both in the number and in functional ability of platelets, the result of which is the occurrence of a hemorrhagic syndrome. Said platelet disorder may be congenital or acquired. In a particular embodiment, said platelet disorder is a congenital platelet disorder. Illustrative, non-limiting examples of congenital platelet disorders include Glanzmann's disease, Bernard Soulier disease, Bolin-Jamieson syndrome, Wiskott-Aldrich syndrome, Paris-Trousseau-Jacobsen syndrome, X chromosome thrombocytopenia, Gray platelet syndrome, Sebastian syndrome and Fanconi anemia. In another particular embodiment, said platelet disorder is an acquired platelet disorder. Illustrative, non-limiting examples of acquired platelet disorders incluye myeloproliferative disorders, such as thrombocythemia, polycythemia, chronic myelocytic leukemia, etc.; there are functional platelet disorders in myeloid metaplasia with increased bleeding time, glass bead retention defects, platelet aggregation defect, abnormal release, and platelet factor III defect. Functional platelet defects have been found in dysproteinemias in scurvy and in congenital heart disease and cirrhosis.

In one embodiment of the seventh aspect of the invention, it is provided the combination or compositions as defined above for use in the topical treatment of hemorrhages by its application to the injury site.

The term "subject" as used herein includes any member of an animal species, including the human species; by way of an illustrative, non-limiting example, said subject can be a mammal, such as a primate, a domestic animal, a rodent, etc. Said subject is preferably a man or woman of any age and race. In a particular embodiment, said subject is a human being with no history of hemostasis disorders, such as an individual having no coagulopathies or platelet disorders. In another particular embodiment, said subject is a human being having a history of hemostasis disorders, such as an individual having hemorrhagic diathesis, for example, a coagulopathy, such as a congenital or acquired coagulopathy, or a platelet disorder, such as a congenital or acquired platelet disorder.

Since TF is a well known pro-angiogenic molecule, thus combinations and compositions of the invention can also be used in the treatment of a disease associated to a deficient angiogenesis.

The term "disease associated to deficient angiogenesis", as used herein, relates to diseases which can be cured by activating vessel formation. The expression "vessel formation" relates to a vessel formation of any kind and at any site. The promotion of vessel formation may be useful in a number of clinical conditions. For example, the combinations of the invention may be used to promote angiogenesis of collateral vasculature in myocardial tissue during or following ischaemic disease, myocardial infarction or following coronary bypass surgery. Other diseases or conditions which may be treated by the provision of the combinations and compositions of the invention include vascular disease and/or ischaemic disease causing pathology of the peripheral or central nervous system. Such conditions/diseases may include cerebrovascular accidents, e.g. caused by clot occlusions or by rupture of aneurysms, or general/localised ischemia causing neuronal death or peripheral functional impairment such as in motor or sensory functions or speech impairment, ischemic cardiomyopathy, or peripheral arterial disease, such as chronic limb ischemia claudication (skeletal muscle), rest pain/ischemic ulceration/gangrene. Moreover, the promotion of vessel formation is adequate for replacing impaired, e g., old, blood vessels. They can be present, e.g., in the brain or heart, so that an apoplexy or infarction can be prevented or treated. Precautions can also be taken against presbyphrenia. In addition, it relates to a vessel formation for treating arteriosclerosis, Crohn's disease and ulcerative colitis, diabetic retinopathy and deep venous thrombosis of the legs/ulcus cruris as well as the prevention of relapses.

Finally, in a further aspect the present invention provides kits comprising the combinations and compositions of the invention together with an applicator.

The kit can take the form of (a) three parts, in such a way that each part contains each of the components forming the combination of the invention, that is one part of the kit contains the thrombin, another one contains the fibrinogen, and the other one contains the lipidated tissue factor; or, alternatively, (b) as two parts, in such a way that two of the components of the combination (either thrombin and lipidated tissue factor or fibrinogen and lipidated tissue factor) shear one part, and the third component is contained, separately, in a second part.

Thus, in one embodiment, the kit comprises: a) a first part comprising thrombin; b) a second part comprising fibrinogen; and c) a third part comprising the recombinant lipidated tissue factor; and d) an applicator to apply simultaneously all the components included in the kit.

In another embodiment, the kit comprises: a) a first part comprising thrombin and lipidated tissue factor; b) a second part comprising fibrinogen; c) an applicator an applicator to apply simultaneously all the components included in the kit.

In still another embodiment, the present invention provides a kit comprising a) a part comprising thrombin; b) a second part comprising fibrinogen and lipidated tissue factor; and c) an applicator an applicator to apply simultaneously all the components included in the kit.

In still another embodiment, the present invention provides a kit comprising a single part containing all thrombin, fibrinogen, and lipidated tissue factor.

The thrombin, fibrinogen, and lipidated tissue factor contained in the kit can be in any suitable form, such as in solution, suspension, or powder, together with the appropriate pharmaceutically or veterinary acceptable excipients and/or carriers.

In addition, the kit can include instructions for its use in any of the applications mentioned above.

In one embodiment, the kit further comprises a cross-linking agent.

In another embodiment, the kit further comprises a fybrinolysis inhibitor.

In still another embodiment, the kit further comprises CaCl₂.

In a further embodiment, the kit further comprises a cross-linking agent and a fybrinolysis inhibitor.

In a further embodiment, the kit comprises a cross-linking agent, fybrinolysis inhibitor, and CaCl₂.

The cross-linking agent, the fybrinolysis inhibitor, and CaCl₂, can be included in any one of the parts identified above for thrombin, fibrinogen, and lipidated tissue factor, or alternatively, each one can be in further separate parts or can be sharing the same part of the kit.

Any further component can be included in the kit provided that it does not negatively effect to the hemostatic and sealant profiles of the combination included therein. Such extra-components can be included in any one of the parts identified above for thrombin, fibrinogen, and lipidated tissue factor. Alternatively, each one of the "extra-components" of the kit can be in further separate parts or can be sharing the same part of the kit.

Preferably, said cross-linking agent is FXIII.

Preferably, said fybrinolysis inhibitor is selected from aprotinin, tranexamic acid, and aminocaproic acid.

Illustrative non limiting examples of applicators that allows to apply simultaneously all the components included in the kit are barreled syringes, spray applicators, or a device for dispensing the components when they are in powder form, among others.

An example of dispensing device of powder is the one disclosed in WO2010/07033.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### Example 1: Production of the recombinant tissue factor based on the expression of the full-length TF his-tag modified protein in yeast hereinafter also referred as "TT-173")

The yeast episomal vector pTT-10301 described in Example 1 of WO2008080989 which comprises the URA3 gene, the ampicillin resistance gene, the yeast 2 µ origin of replication, the glyceraldehyde-3-phosphate dehydrogenase (GPD) promoter and the yeast transcription termination signal of the phosphoglycerate kinase, was used to clone, under the control of the GPD promoter, a cDNA of SEQ ID NO: 6 coding for the mature hTF protein (aa 33-295 of SEQ ID NO:1) with the His-tag at the 3' end and an Asn124Ala mutation which inactivates one of the potential N-glycosylation sites in the native hTF sequence (SEQ ID NO: 5).

SEQ ID NO: 6 was obtained as follows. The human cDNA sequence of TF (Genbank accession number BC011029, SEQ ID NO: 11) was amplified using the primers of sequence SEQ ID NO: 7 and SEQ ID NO: 8. The resulting amplified DNA sequence was further modified by site-directed mutagenesis, using primers of sequence SEQ ID NO: 9 and SEQ ID NO: 10. For this, the Quick Change site-directed mutagenesis kit (Stratagene) was used, following the method described in Stratagene manuals. With such latter mutagenesis, the TF sequence containing the Asn to Ala point mutation at amino acid residue 124 of the TF protein sequence was generated.

After transformation, yeast strains capable of growing in uracil-free media were collected and tested for their ability to express hTF by Western-blot analysis of the yeast extracts essentially as described in WO2008080989. Briefly, yeasts, containing the episomal expression plasmid with a modified version of the TF coding gene (SEQ ID NO: 6), are grown in a fermentor, and the resulting cells collected by centrifugation. The collected cells are resuspended in an appropriate lysis buffer (20 mM phosphate, 50 mM NaCl, pH 7.4), and subjected to high pressure lysis by passing three times through a homogenizer at 1000 bars of pressure to obtain the fermentation lysate (fermentation homogenate). The cell suspension is maintained cold by sumerging the container in an ice bath. After that, the heaviest material is eliminated from the fermentation lysate by means of centrifugation, thus obtaining a clarified yeast extract (CYE) supernatant. The CYE obtained from the homogenization step is diluted with lysis buffer until total protein content reach a concentration between 5-6 mg/mL, and filtered through consecutive filters of 0.45 µm, 0.2 µm, and 0.1 µm respectively to reduce the amount of non-membrane host cell derived proteins (HCP) and to obtain a more homogeneous material in rTF-vesicles.

The vesicles in the microfiltrate retentate of 0.1 µm have diameters below 0.2 µm. To eliminate traces of DNA, the microfiltrate retentate of 0.1 µm is then treated with 10 U/mL of the endonuclease Benzonase (Merck) and magnesium chloride to a final concentration of 1 mM, incubated for 12 h at 4°C in a glass container rocking at approximately 20 rpm, and used for further enrichment steps.

To further fractionate different vesicle subpopulations according to their size, and to remove unwanted residual cell material, the filter-clarified yeast extract is applied to a Sephacryl size-exclusion chromatography column. After separation, fractions 2 to 22, corresponding to vesicles enriched in rTF, are pooled and filter-sterilized. After that, phosphatydylserine (PS) at a final concentration of 0.1 mg/mL is added to the sterilized product and the mixture is maintained at room temperature for 2 h to allow the incorporation of PS into the vesicles. As a previous step, PS could be prepared as multilamelar vesicles by resuspension of lipids in a buffered solution followed by sonication, following the Morrissey Lab Protocol for Preparing Phospholipid Vesicles (SUV) by sonication. Briefly: (1) Dispense 2.6 µmole total phospholipids (PL) in a glass test tube (a 13 x 100 mm tube is a convenient size); (2) In the fume hood, dry the PL mixture under a gentle stream of nitrogen or argon. When dry, speed-vac for an additional 60 minutes under high vacuum. (This is to remove any residual chloroform.); (3) To the dried-down PL, add 2.6 ml room temperature HBS solution and cover the end of the tube with parafilm. Let sit 1 hr at room temperature; (4) Vortex tube vigorously to completely resuspend the PL. The result should be a milky, uniform suspension; (5) Fill the bath sonicator with room temperature water. Using a ring stand and test tube clamp, suspend the tube containing the PL suspension in the bath. The liquid level inside the tube should equal that outside the tube. Sonicate until the suspension changes from milky to nearly clear (i.e., only very slightly hazy) in appearance. Check every 10 min; it will usually take between 10 and 30 min total sonication time. (Be sure not to let the bath overheat, and do not drain the bath until it has completely cooled.); and (6) Store the final product at 4 degrees C. The result is a suspension of small unilamellar vesicles (SUV) containing a total of 1 mM phospholipid in HBS.

After 2 h of incubation, the sterile product, containing PS enriched vesicles, is aliquoted and lyophilized. This lyophilized material corresponds to the rTF used in the tests below and consists of a protein of SEQ ID NO:5 anchored to a lipidic vesicle highly enriched in PS.

### Example 2: Effect of the addition of TF over FS on the propagation of the clot in normal plasma and in heparinized plasma

The FS used as reference in this test is the one marketed as Tissucol®. 1 mL of Tissucol ® contains: 500U of human thrombin, 115 mg of fibrinogen, aprotinin 3000 U, and Calcium Chloride 40 µmol.

The combination of the invention was made by adding to 2 mL of Tissucol and 300 ng of the lipidated TF obtained following Example 1.

For these studies, 2.5 mL of normal plasma (pooled plasma from five healthy donors) were placed in a 4 cm long lane. Then, 1 mL of the sealant combination to be tested, either the reference (Tissucol ®), or the one of the present invention (Tissucol ® plus lipidated TF), was applied over one of the ends.

Every 15 seconds after test item application, for 5 minutes, the degree of coagulation, 3 cm away from the site of application,was determined with the aid of a espectrophotometer. For this, the optical density of plasma was measured with a spectrophotometer (Biotek) at 340 nm . FIG. 1 shows the coagulation percentage of FS reference (Tissucol ®) (grey square), and of the combination of the invention (FS +TF) (black triangle), at different times after the application of each product, 3 cm away from the site of application.

As it is shown, the combination of FS + TF has already provided 50% of coagulation in normal plasma after 105 seconds of the application (FIG. 1A). However, when the FS is administered alone, it is only at this time (105 seconds), when coagulation starts. These differences are more pronounced when a similar experiment is carried out with heparin (Clexane ®) -treated plasma at both limits of its therapeutic concentration: 0.5 U/mL (FIG.1 B) and 1U/mL (FIG. 1 C).

From these results it can be concluded that addition of lipidated TF to a FS comprising FIIa and FI, improves the hemostatic profile through the propagation of the clot.

### Example 3: In vivo hemostatic effect enhancement of a FS after the addtition of lipidated TF

The assay performed is the one disclosed by Kemal Karakaya et al in The Journal of Trauma Injury, Infection, and Critical Care, 2000, vol. 49 (2), p. 246-250.

Briefly, fifty-two male (HsdHan: WIST, Harlan Interfauna) rats (380-400 gm) were used in this study. The animals were housed in a climate-controlled facility (cages: 25x50 cm. Two animals per cage). Food (Global Diet 2014, Harlan Tekland) and water was available ad libitum. Animals were maintained in the University of Barcelona. Faculty of Biology. Department of Biochemistry and Molecular Biology. ExperimentalAnimalsResearch Unit (CEREMET). The study protocol was approved by the Ethic Committee of Experimental Animals (CEEA) from the University of Baecelona, and the Department of Agriculture, Food, and Environment of the Generalitat of Catalonia. Reference of approval: DAAM 5996. All the animals received humane care in accordance with the requirements of the UE Animal Welfare Regulations.

The animals were allocated randomly into three groups to receive either: No treatment (n= 15), Tissucol ® (n=21), or Tissucol ® plus lipidated TF (200 ng/mL) obtained following Example 1 (Tissucol ® +TF, n=16).

The animals were anesthetized with an intramuscular injection of 100 mg/kg ketamin. After performing a midline laparotomy, the abdominal cavity was wiped dry with cotton sponges. A portion of the liver was sharply excised by scissors. As a rapidly bleeding liver surface was easily visualized, no manipulation to the bleeding surface was done.The resected surface was then either treated with Tissucol ®, Tissucol ® plus lipidated TF, or left untreated according to randomization.After that, blood was allowed to accumulate in the peritoneal cavity in all groups, and it was collected with small cotton sponges in order to avoid spillage out of the abdominal cavity. At the end of the study period, shed blood in the abdominal cavity was collected with small cotton sponges. The total blood loss was calculated as blood soaked cotton sponges minus the weight of preweighed dry cotton sponges used for each animal. The animals were monitored for 30 min after the liver injury.

As shown in FIG.2, Tissucol ® (FS) application results in a reduction of blood loss when compared with untreated animals. However, the addition of lipidated TF to Tissucol ® (FS) produces an increase of approximately 50% in the efficacy in respect of FS alone.

The statistically significance of the results were confirmed by the Newman-Keuls Multiple Comparison test, given the following p values: Tissucol ® vs Untreated: p value <0.001, Tissucol ® vs Tissucol ® +lipidated TF: p value <0.01, and Tissucol ® +lipidated TF vs Untretaed: p value <0.001.

### Example 4: Consistency of the clot

Each of the reference FS and the combination used in Example 2 were applied over one of the ends of a 4 cm long lane containing 2.5 mL of heparin (Clexane ®) -treated plasma.

When applied the combination of the invention it was observed that it spread substantially faster through the lane than the reference sealant. In addition, with the help of a tip pipette, the clots obtained with each of the combinations were removed: the clot obtained with the commercial FS was fragile and easily broken, whereas the clot resulting from the application of the combination of the invention did not. This was indicative that the clot obtained with the combination of the invention had a much higher consistency than the clot obtained with the reference Tissucol®. The fact that the clot obtain with the combination of the invention showed a much better consistency with respect to the one obtained with the commercial reference (Tissucol®) means that it is substantially reduced the risk of clot breakage associated to the seals derived from commercial FSs and, therefore, it is absolutely effective in sealing the injury/wound.

Without being bound to the theory, the present inventors believe that the tissue factor is embedded in the fibrin network formed (due to the thrombin and fibrinogen included in the combination) and that due to this distribution of the tissue factor within the fibrin network, an expansion effect of the clot occurs, giving rise to the efficiently sealed of the injury or lesion.

### Example 5: hemostatic and sealant profile of thrombin-free sealant compositions including lipidated tissue factor.

In an attempt to determine the hemostatic and sealant properties of the compositions disclosed in WO97/29792 some free-thrombin sealant compositions were prepared and analyzed by TEM following manufacturer recommendations (ROTEM® instruction manual). The parameters MCF (Maximal clot firmness) and G (Elastic Modulus Strength), related to the global strength of the clot were recorded.

To compare the strength of the clot from those compositions, with the clot induced by FSs containing the same parameters were also determined for a commercial FS (Tissuol) and in a non-commercial FS.

The samples tested were:
**1:** FII, FV, FVII, FX, FXIII, FI (source: Pooled plasma obtained from five different healthy donors)+ lipidated TF 200 ng/mL (source: TT-173) + Calcium Chloride 5 mM.
**2:** FII, FV, FVII, FX, FXIII, FI (source: Pooled plasma obtained from five different healthy donors)+ lipidated TF 200 ng/mL (source: TT-173) + FI 3 mg/mL (source SIGMA. ref. F4129) + Calcium Chloride 5 mM
**3:** FII, FV, FVII, FX, FXIII, FI (source: Pooled plasma obtained from five different healthy donors)+ lipidated TF 200 ng/mL (American Diagnostica ref. 4500L) + Calcium Chloride 5 mM
**4:** FII, FV, FVII, FX, FXIII, FI (source: Pooled plasma obtained from five different healthy donors)+ lipidated TF 200 ng/mL (American Diagnostica ref. 4500L) + FI 3 mg/mL (source SIGMA. ref. F4129) + Calcium Chloride 5 mM
**5:** FII: 100 µg/mL, FV: 10 µg/mL, FVII: 0.5 µg/mL , FX: 8 µg/mL, FXIII: 10µg/mL, FI: 6 mg/mL (source: FI (ref. F4129 SIGMA), FII: (ref. HCP-0010 CellSystems), FXIII: (ref. HCXIII-0160 CellSystems), FX (ref. HCX-0050 CellSystems), FV (ref. HCV-0100 CellSystems), FVII (ref. HCVII 0030 CellSystems)) + lipidated TF 200 ng/mL (source: TT-173) + Calcium Chloride 5 mM. The mixture of the components was prepared freshly 10 min before their use.
**6:** FII: 100 µg/mL, FV: 10 µg/mL, FVIIa: 0.5 µg/mL , FX: 8 µg/mL, FXIII: 10µg/mL, FI: 6 mg/mL (source: FI (ref. F4129 SIGMA), FIIa: (ref. HCP-0010 CellSystems), FXIII: (ref. HCXIII-0160 CellSystems), FX (ref. HCX-0050 CellSystems), FV (ref. HCV-0100 CellSystems), FVII (ref. HCVII 0031 CellSystems)) + lipidated TF 200 ng/mL (source: TT-173) + Calcium Chloride 5 mM. The mixture of the components was prepared freshly 10 min before their use.

MCF and G values were also determined for two FS compositions: commercialTissucol® and non-commercial FS:
Non-commercial FS, composition per mL:
   Fibrinogen (FI) (ref. F4129. SIGMA): 115 mg
   Thrombin (FIIa) (ref. T4648 SIGMA): 500 IU
   Calcium chloride: 40 µmol

The results, corresponding to the analyses of 400 µL of each combination are shown in Table I (n=3):

**TABLE I**

| | 1 | 2 | 3 | 4 | 5 | 6 | Tissucol® | FS |
|---|---|---|---|---|---|---|---|---|
| MCF (mm) | 29 | 42 | 32 | 40 | 4 | 9 | 94.3 | 83 |
| G (Kdyn/cm²) | 2 | 3.6 | 2.3 | 3.3 | 0.2 | 0.5 | 90 | 24 |

From the data summarized in Table I, one can see that the inclusion of lipidated tissue factor to thrombin-free sealant compositions provides sealant compositions with MCF (maximal clot firmness) and G (elastic modulus strength) values substantially lower than those of the commercial thrombin sealant compositions. In particular, the MCF is at least 200% lower than the MCF of the reference compositions. On the other hand, the G value is at least about 2000% lower than those of the commercial thrombin sealant compositions.

As the skilled person in the art knows, as low the MCF and G values are as worse is the sealant profile of the clot. Therefore, by adding lipidated tissue factor to thrombin-free compositions no improvement in the sealant activity is achieved. And, therefore, the sealant effect found by the present inventors when lipidated tissue factor is added to thrombin sealant compositions cannot be derived from the teachings of WO97/29792.

### Example 6: Analysis of the coagulation time

Other parameter relevant for the analyses of the efficacy of a FS is the time required for their different components to create a stable clot. This can be easily quantitated by the aid of a coagulometer. A ternary composition corresponding to the present invention FS+TT-173 (Fibrinogen (FI) (ref. F4129. SIGMA): 115 mg/mL, Thrombin (FIIa) (ref. T4648 SIGMA): 500 IU/mL, TT-173 (200 ng TF/mL), 40 µmol/mL calcium chloride), was also included in the assay.

Coagulometric analyses were carried out as follows: Aliquots (200 µL) from the compositions tested in Example 5 or FS+TT-173, were added to cuvettes containing a stainless steel sphere. A magnetic stirred incorporated into the coagulometer (Diagnostica Stago, Inc. NJ, USA) moves the steel sphere until it stop because of the clot. This time called coagulation time (in seconds) was recorded. The experiment was stopped after 300 seconds. Results are shown in Table II.

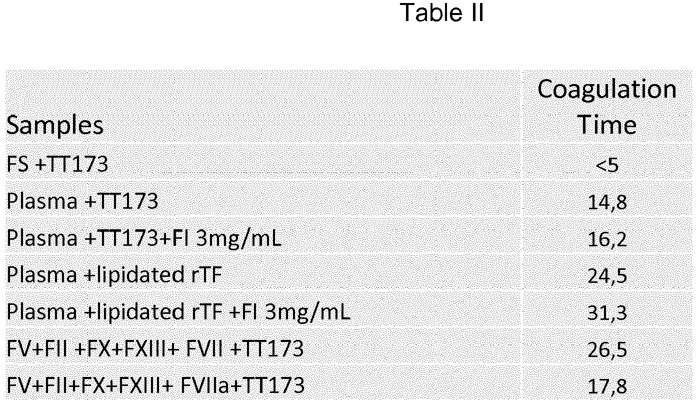

As can be seen in Table II the coagulation time induced by the FS containing FIIa, show a coagulation time below 5 seconds, whereas the combinations of WO97/29792 needed between 15 and 31 seconds, i.e, at least 300% more time, to indiuced enough Fibrin fibers to be detected by the coagulometer.

In view of the above data we can conclude that the ternary FS composition described in the invention shows a fast and strong clot than the compositions described in WO97/29792.

### Example 7: hemostatic capability of the combination of the invention in heparinized plasma samples.

A clot produced by:
(a) 30 µL of a non-commercial FS (see composition under Example 5);
(b) 30 µL of a ternary combination FS+TT173 (see composition under Example 6) or
(c) 30 µL of a similar ternary combination but containing lipidated rTF (200 ng/mL. source American Diagnostica ref.4500L) instead TT-173 as source of TF.
was added to 300 µL of heparinized plasma (0.6 U/mL). Next, Clot Time (CT= time from the application of the clot until the appearance of the first fibrin nets), Clot Formation Time (CFT= time from the formation of the first fibrin nets until the formation of the whole clot), and Maximum Clot Formation (MCF= clot firmness) parameters were determined by TEM, following the manufacturer recommendations (ROTEM® instruction manual).

The results are summarized in Table III below:

**Table III**

| | FS | FS+TT-173 | FS+rTF |
|---|---|---|---|
| CT | 171.6 | 41.3 | 37.5 |
| CFT | -- | 159.6 | 356.5 |
| MCF | 7 | 33.6 | 34.5 |

It is remarkable the fact that when FS alone was used, no stable clot was obtained in the assay. In fact, after 40 minutes (which was the time spent in the assay) only the clot formation onset was detected (which required 171 seconds).

On the contrary, with the inclusion of lipidated tissue factor, the ternary combination started the coagulation sooner and, in both cases, a stable clot was generated, said clot showing a high consistency (MCF value).

The fact that the ternary combination of the invention is able of generating stable consistent clots in so short period of time is of great importance in critical medical scenarios.

### REFERENCES CITED IN THE APPLICATION

WO2008080989;
WO2010/07033;
WO2011131658;
WO97/29792;
Mimms L. T. et al., "Phospholipid vesicle formation and transmembrane protein incorporation using octyl glucoside", Biochemistry, 1981, vol. 20(4), p. 833-840; and
Kemal Karakaya et al., "Evaluation of a New Hemostatic Agent Ankaferd Blood Stopper in Experimental Liver laceration" , The Journal of Trauma Injury, Infection, and Critical Care, 2000, vol. 49 (2), p. 246-250.

### SEQUENCE LISTING

<110> Thrombotargets Europe SL
<120> Sealant composition
<130> P2530EP00
<150> EP12198839.8
   <151> 2012-12-21
<160> 11
<170> BiSSAP 1.0
<210> 1
   <211> 263
   <212> PRT
   <213> Homo sapiens
<220>
   <221> SOURCE
   <222> 1..263
   <223> /mol_type="protein"
   /organism="Homo sapiens"
<400> 1
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
<210> 2
   <211> 6
   <212> PRT
   <213> artificial sequences
<220>
   <221> SOURCE
   <222> 1..6
   <223> /mol_type="protein"
   /note="epitope AHGHRP"
   /organism="artificial sequences"
<400> 2
   Ala His Gly His Arg Pro
   1               5
<210> 3
   <211> 14
   <212> PRT
   <213> artificial sequences
<220>
   <221> SOURCE
   <222> 1..14
   <223> /mol_type="protein"
   /note="Epitope"
   /organism="artificial sequences"
<400> 3
   Pro Ile His Asp His Asp His Pro His Leu Val Ile His Ser
   1               5                   10
<210> 4
   <211> 7
   <212> PRT
   <213> artificial sequences
<220>
   <221> SOURCE
   <222> 1..7
   <223> /mol_type="protein"
   /note="X can be any naturally occurring amino acid"
   /organism="artificial sequences"
<400> 4
   Gly Met Thr Cys Xaa Xaa Cys
   1               5
<210> 5
   <211> 269
   <212> PRT
   <213> artificial sequences
<220>
   <221> SOURCE
   <222> 1..269
   <223> /mol_type="protein"
   /note="Human mature TF with N124A mutation and C terminal hexahis tidine "
   /organism="artificial sequences"
<400> 5
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Ala Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser His His His His His His
               260                 265
<210> 6
   <211> 813
   <212> DNA
   <213> artificial sequences
<220>
   <221> source
   <222> 1..813
   <223> /mol_type="DNA"
   /note="DNA encoding human mature TF with N124A and C-terminal "
   /organism="artificial sequences"
<400> 6
   atgtcaggca ctacaaatac tgtggcagca tataatttaa cttggaaatc aactaatttc      60
   aagacaattt tggagtggga acccaaaccc gtcaatcaag tctacactgt tcaaataagc     120
   actaagtcag gagattggaa aagcaaatgc ttttacacaa cagacacaga gtgtgacctc     180
   accgacgaga ttgtgaagga tgtgaagcag acgtacttgg cacgggtctt ctcctacccg     240
   gcagggaatg tggagagcac cggttctgct ggggagcctc tgtatgagaa ctccccagag     300
   ttcacacctt acctggagac aaacctcgga cagccaacaa ttcagagttt tgaacaggtg     360
   ggaacaaaag tggcagtgac cgtagaagat gaacggactt tagtcagaag gaacaacact     420
   ttcctaagcc tccgggatgt ttttggcaag gacttaattt atacacttta ttattggaaa     480
   tcttcaagtt caggaaagaa aacagccaaa acaaacacta atgagttttt gattgatgtg     540
   gataaaggag aaaactactg tttcagtgtt caagcagtga ttccctcccg aacagttaac     600
   cggaagagta cagacagccc ggtagagtgt atgggccagg agaaagggga attcagagaa     660
   atattctaca tcattggagc tgtggtattt gtggtcatca tccttgtcat catcctggct     720
   atatctctac acaagtgtag aaaggcagga gtggggcaga gctggaagga gaactcccca     780
   ctgaatgttt cacatcacca tcaccatcac tag                                  813
<210> 7
   <211> 33
   <212> DNA
   <213> artificial sequences
<220>
   <221> source
   <222> 1..33
   <223> /mol_type="DNA"
   /note="upstream primer encoding the first amino acids of mature T F lacking the signal peptide and containing an initiation codon A TG in frame with TF ORF), and a restriction BamHI site" /organism="artificial sequences"
<400> 7
   gggatccgca tgtcaggcac tacaaatact gtg 33
<210> 8
   <211> 50
   <212> DNA
   <213> artificial sequences
<220>
   <221> source
   <222> 1..50
   <223> /mol_type="DNA"
   /note="downstream primer containing the histidine coding nucleoti des and a new termination signal TAG, both containing a restrict ion BamHI site"
   /organism="artificial sequences"
<400> 8
   cggatccgtg tcgacctagt gatggtgatg gtgatgtgaa acattcagtg 50
<210> 9
   <211> 33
   <212> DNA
   <213> artificial sequences
<220>
   <221> source
   <222> 1..33
   <223> /mol_type="DNA"
   /note="primer"
   /organism="artificial sequences"
<400> 9
   gtgggaacaa aagtggcagt gaccgtagaa gat 33
<210> 10
   <211> 27
   <212> DNA
   <213> artificial sequences
<220>
   <221> source
   <222> 1..27
   <223> /mol_type="DNA"
   /note="primer"
   /organism="artificial sequences"
<400> 10
   atcttctacg gtcactgcca cttttgt 27
<210> 11
   <211> 1374
   <212> DNA
   <213> Homo sapiens
<220>
   <221> source
   <222> 1..1374
   <223> /mol_type="DNA"
   /organism="Homo sapiens"
<400> 11
   gggggcgccg ccggcccttt atagcgcgcg gggcaccggc tccccaagac tgcgagctcc      60
   ccgcaccccc tcgcactccc tctggccggc ccagggcgcc ttcagcccaa cctccccagc     120
   cccacgggcg ccacggaacc cgctcgatct cgccgccaac tggtagacat ggagacccct     180
   gcctggcccc gggtcccgcg ccccgagacc gccgtcgctc ggacgctcct gctcggctgg     240
   gtcttcgccc aggtggccgg cgcttcaggc actacaaata ctgtggcagc atataattta     300
   acttggaaat caactaattt caagacaatt ttggagtggg aacccaaacc cgtcaatcaa     360
   gtctacactg ttcaaataag cactaagtca ggagattgga aaagcaaatg cttttacaca     420
   acagacacag agtgtgacct caccgacgag attgtgaagg atgtgaagca gacgtacttg     480
   gcacgggtct tctcctaccc ggcagggaat gtggagagca ccggttctgc tggggagcct     540
   ctgtatgaga actccccaga gttcacacct tacctggaga caaacctcgg acagccaaca     600
   attcagagtt ttgaacaggt gggaacaaaa gtgaatgtga ccgtagaaga tgaacggact     660
   ttagtcagaa ggaacaacac tttcctaagc ctccgggatg tttttggcaa ggacttaatt     720
   tatacacttt attattggaa atcttcaagt tcaggaaaga aaacagccaa aacaaacact     780
   aatgagtttt tgattgatgt ggataaagga gaaaactact gtttcagtgt tcaagcagtg     840
   attccctccc gaacagttaa ccggaagagt acagacagcc cggtagagtg tatgggccag     900
   gagaaagggg aattcagaga aatattctac atcattggag ctgtggtatt tgtggtcatc     960
   atccttgtca tcatcctggc tatatctcta cacaagtgta gaaaggcagg agtggggcag    1020
   agctggaagg agaactcccc actgaatgtt tcataaagga agcactgttg gagctactgc    1080
   aaatgctata ttgcactgtg accgagaact tttaagagga tagaatacat ggaaacgcaa    1140
   atgagtattt cggagcatga agaccctgga gttcaaaaaa ctcttgatat gacctgttat    1200
   taccattagc attctggttt tgacatcagc attagtcact ttgaaatgta acaaatggta    1260
   ctacaaccaa ttccaagttt taatttttaa caccatggca ccttttgcac ataacatgct    1320
   ttagattata tattccgccg tcccaaaaaa aaaaaaaaaa aaaaaaaaaa aaaa          1374

## Claims

1. A combination comprising a lipidated tissue factor, thrombin, and fibrinogen.

2. The combination according to claim 1, wherein at least one of the N-glycosilation site of the tissue factor is non-functional.

3. The combination according to any one of the claims 1-2, wherein said tissue factor is a fusion protein comprising a first portion which comprises the tissue factor protein, and a second portion which comprises another peptide or protein.

4. The combination according to claim 3, wherein the second portion is a tag.

5. The combination according to any one of the claims 1-4, wherein the fusion protein has a first portion which comprises the mature human tissue factor protein with at least one of the N-glycosilation sites being non-functional, and a second portion which comprises a His-tag.

6. The combination according to claim 5, wherein the fusion protein corresponds to SEQ ID NO: 5.

7. The combination according to claim 6, wherein the lipidated TF is inserted in a microvesicle.

8. The combination according to any one of the claims 1-14, wherein the thrombin is human thrombin.

9. The combination according to any one of the claims 1-15, wherein the fibrinogen is human fibrinogen.

10. A sealant composition comprising the combination according to any one of the claims 1-9, and a material carrier.

11. A pharmaceutical composition comprising a therapeutically effective amount of the combination as defined in any one of the claims 1-9, together with other appropriate pharmaceutically or veterinary acceptable excipients and/or carriers.

12. A combination according to any one of the claims 1-9 or the composition according to claim 10 for use as sealant agent.

13. The combination according to any one of the claims 1-9 or the composition according to claim 10-11 for use as a medicine.

14. A combination according to any one of the claims 1-9 or a composition according to any one of the claims 10-11 for use in treatment of haemorrhages.

15. A kit comprising the combination as defined in any one of the claims 1-9 and an applicator that allows the simultaneous application of all the components forming the combination.

## Patentansprüche

1. Eine Kombination umfassend einen lipidierten Gewebefaktor, Thrombin, und Fibrinogen.

2. Die Kombination nach Anspruch 1, wobei mindestens eine der N-Glykolisierungsstellen des Gewebefaktors nicht-funktionell ist.

3. Die Kombination nach einem der Ansprüche 1-2, wobei der Gewebefaktor ein Fusionsprotein umfassend einen ersten Teil, der das Gewebefaktorprotein umfasst, und einen zweiten Teil, der ein anderes Peptid oder Protein umfasst, ist.

4. Die Kombination nach Anspruch 3, wobei der zweite Teil ein Tag ist.

5. Die Kombination nach einem der Ansprüche 1-4, wobei das Fusionsprotein einen ersten Teil hat, der das reife menschliche Gewebefaktorprotein umfasst, wobei mindestens eine der N-Glykolisierungsstellen nicht-funktionell ist, und einen zweiten Teil hat, der ein His-Tag umfasst.

6. Die Kombination nach Anspruch 5, wobei das Fusionsprotein der SEQ ID NO: 5 entspricht.

7. Die Kombination nach Anspruch 6, wobei der lipidierte GF in einer Mikrovesikel eingesetzt ist.

8. Die Kombination nach einem der Ansprüche 1-14, wobei das Thrombin menschliches Thrombin ist.

9. Die Kombination nach einem der Ansprüche 1-15, wobei das Fibrinogen menschliches Fibrinogen ist.

10. Eine Dichtungsmittelzusammensetzung umfassend die Kombination nach einem der Ansprüche 1-9 und einen Materialträger.

11. Eine pharmazeutische Zusammensetzung umfassend eine therapeutisch wirksame Menge der Kombination wie in einem der Ansprüche 1-9 definiert zusammen mit anderen geeigneten pharmazeutischen oder tiermedizinischen akzeptablen Hilfsstoffen und/oder Trägersubstanzen.

12. Eine Kombination nach einem der Ansprüche 1-9 oder die Zusammensetzung nach Anspruch 10 zur Verwendung als Dichtungsmittel.

13. Die Kombination nach einem der Ansprüche 1-9 oder die Zusammensetzung nach Anspruch 10-11 zur Verwendung als Arzneimittel.

14. Eine Kombination nach einem der Ansprüche 1-9 oder eine Zusammensetzung nach einem der Ansprüche 10-11 zur Verwendung in der Behandlung von Hämorrhagien.

15. Ein Kit umfassend die Kombination wie in einem der Ansprüche 1-9 definiert und einen Applikator, der die gleichzeitige Applikation von allen Bestandsteilen ermöglicht, welche die Kombination ausmachen.

## Revendications

1. Une combinaison comprenant un facteur tissulaire lipidé, de la thrombine et du fibrinogène.

2. La combinaison selon la revendication 1, dans laquelle au moins un des sites de N-glycolysation du facteur tissulaire est non fonctionnel.

3. La combinaison selon l'une quelconque des revendications 1-2, dans laquelle ledit facteur tissulaire est une protéine de fusion comprenant une première partie qui comprend la protéine du facteur tissulaire, et une deuxième partie que comprend un autre peptide ou protéine.

4. La combinaison selon la revendication 3, dans laquelle la deuxième partie est un tag.

5. La combinaison selon l'une quelconque des revendications 1-4, dans laquelle la protéine de fusion a une première partie que comprend la protéine du facteur tissulaire humaine mature étant au moins un des sites de N-glycolysation non fonctionnel, et une deuxième partie qui comprend un His-tag.

6. La combinaison selon la revendication 5, dans laquelle la protéine de fusion correspond à la SEQ ID NO : 5.

7. La combinaison selon la revendication 6, dans laquelle le FT lipidé est inséré dans une microvésicule.

8. La combinaison selon l'une quelconque des revendications 1-14, dans laquelle la thrombine est thrombine humaine.

9. La combinaison selon l'une quelconque des revendications 1-15, dans laquelle le fibrinogène est fibrinogène humain.

10. Une composition d'étanchéité comprenant la combinaison selon l'une quelconque des revendications 1-9, et un support matériel.

11. Une composition pharmaceutique comprenant une quantité thérapeutiquement efficace de la combinaison telle que définie dans l'une quelconque des revendications 1-9, avec d'autres excipients et/ou porteurs pharmaceutiques ou vétérinaires appropriés.

12. Une combinaison selon l'une quelconque des revendications 1-9 ou la composition selon la revendication 10, pour l'utilisation comme agent d'étanchéité.

13. La combinaison selon l'une quelconque des revendications 1-9 ou la composition selon la revendication 10-11 pour l'utilisation comme médicament.

14. Une combinaison selon l'une quelconque des revendications 1-9 ou une composition selon l'une quelconque des revendications 10-11 pour l'utilisation dans le traitement d'hémorragies.

15. Un kit comprenant la combinaison telle que définie dans l'une quelconque des revendications 1-9 et un applicateur qui permet l'application simultanée de tous les composants formant la combinaison.
